# EUROPÄISCHE PATENTANMELDUNG

(11) **EP 4 678 757 A1**
(43) Veröffentlichungstag der Anmeldung: **14.01.2026**
(21) Anmeldenummer: 24188318.0
(22) Anmeldetag: 12.07.2024
(51) Int. Cl.: C12P 7/18, C12M 1/00, C12N 9/04, C12P 15/00, C12P 19/02

(54) **VERFAHREN ZUR ENZYMATISCHEN REDUKTION EINER ORGANISCHEN KETO-VERBINDUNG**

(71) Anmelder: Annikki GmbH, 8074 Raaba-Grambach (AT)
(72) Erfinder: STAUNIG, Nicole, 8074 Raaba-Grambach (AT)
(74) Vertreter: Schwarz & Partner Patentanwälte GmbH

(57) **Zusammenfassung**

Verfahren zur enzymatischen Reduktion einer organischen Keto-Verbindung zu einer organischen Hydroxy-Verbindung, wobei NAD(P)H als Kofaktor der enzymatischen Reduktion eingesetzt wird und das bei der Reduktion entstehende oxidierte Kofaktor NAD(P)⁺ enzymatisch mit 2-Propanol als Kosubstrat unter Bildung von Aceton zu NAD(P)H reduziert und Aceton durch Verdampfung abgetrennt wird, wobei die Verdampfung von Aceton in einer Dünnschichtbehandlungsvorrichtung vorgenommen wird.

## Beschreibung

Die Erfindung betrifft ein Verfahren zur enzymatischen Reduktion einer organischen Keto-Verbindung zu einer organischen Hydroxy-Verbindung, wobei NAD(P)H als Kofaktor bei der Reduktion eingesetzt wird und der bei der Reduktion entstandene oxidierte Kofaktor NAD(P)⁺ enzymatisch mit 2-Propanol als Kosubstrat unter Bildung von Aceton zu NAD(P)H reduziert und Aceton durch Verdampfung abgetrennt wird. Die Erfindung betrifft ferner eine Anlage zur Durchführung des Verfahrens.

### Hintergrund der Erfindung

Im Gegensatz zu klassischen chemisch-katalytischen Reaktionen können biokatalytische Reaktionen entweder *in vivo* (Fermentationen, Ganzzell-Verfahren) oder *in vitro* (enzymatisch in zell-freien Systemen) im Allgemeinen unter milden Reaktionsbedingungen (wässeriges Milieu, Umgebungstemperatur, Umgebungsdruck) durchgeführt werden. Darüber hinaus sind die verwendeten Biokatalysatoren (wie Zellen oder Enzyme) nicht-toxisch und bioabbaubar.

Eine wichtige Klasse von Enzymen sind Oxidoreduktasen, die Reduktionen und Oxidationen katalysieren. Ungefähr 50% aller Oxidoreduktasen nutzen Nicotinamid-Adenin-Dinucleotid-Kofaktoren (NAD⁺/NADH oder NADP⁺/NADPH; im Nachfolgenden allgemein zusammengefasst als NAD(P)) als Redox-Kofaktoren (Sellés Vidal et al., 2018).

Aufgrund der hohen Kosten von NAD(P) werden die Redox-Kofaktoren in enzymatisch katalysierten Reaktionen (*in vitro*) nicht in stöchiometrischen Mengen, sondern in katalytischen Mengen eingesetzt. Um einen möglichst hohen Umsatz des Substrats zum Produkt zu ermöglichen, müssen die von den Reaktionsenzymen benötigten Kofaktoren (NAD(P)⁺ oder NAD(P)H) wieder regeneriert werden.

Die Regeneration der Kofaktoren kann zum einen chemisch (heterogen-katalytisch, elektrochemisch, photokatalytisch), aber natürlich auch enzymatisch durchgeführt werden (Chenault et al., 1988; Wang et al., 2017).

Um NAD(P)H aus NAD(P)⁺ enzymatisch zu regenerieren, können beispielsweise die Formiat-Dehydrogenase (Formiat --> CO₂) oder die Glucose-Dehydrogenase (D-Glucose → D-Gluconat) eingesetzt werden (Chenault et al., 1988; Wang et al., 2017).

Eine weitere Methode ist der Einsatz einer Alkoholdehydrogenase (ADH) und einem oxidierbaren Alkohol wie 2-Propanol (Isopropanol) als Hydrid-Donor, aus dem durch Oxidation Aceton gebildet wird. Durch den niedrigeren Siedepunkt von Aceton wird die Entfernung erleichtert, jedoch sind große Mengen an 2-Propanol notwendig, um das Gleichgewicht zur Produktseite zu verschieben (Wang et al., 2017). Alternativ kann die Verschiebung des Gleichgewichts auch durch Verdampfen des flüchtigen Acetons bewerkstelligt werden (US 10113192 B2).

Die Interkonversion von Keto- und Hydroxy-Gruppen durch Redoxreaktionen spielt vor allem in der Kohlenhydratchemie eine wichtige Rolle. Hier bieten sich vor allem Enzyme aufgrund ihrer hohen Regio- und Stereoselektivität als Katalysatoren an.

Die Reduktion der Keto-Gruppe in D-Fructose führt zu zwei Diastereomeren - D-Sorbitol und D-Mannitol.

D-Mannitol weist Anwendungen als nicht-kariogenes und kalorienarmes Süßungsmittel (für Diabetiker-Nahrung), Pharmazeutikum (Diuretikum, Behandlung von Nierenversagen oder als Hilfsstoff in Tabletten und Inhalern) für die Produktion von Spezialchemikalien auf (Saha et al., 2011; Bhatt et al., 2013; Chen et al., 2020).

Da die natürlichen Vorkommen beispielsweise in Früchten und Gemüse, aber auch in Meeresalgen den steigenden Bedarf an D-Mannitol nicht decken können (Saha et al., 2011), wurden und werden Verfahren zur synthetischen Herstellung von D-Mannitol entwickelt.

Mehr als 50 000 Tonnen D-Mannitol pro Jahr werden durch Hydrierung eines Sirups bestehend aus 50% D-Fructose sowie 50% D-Glucose bei hohen Drücken und Temperaturen an einem Raney-NickelKatalysator hergestellt. Das erhaltene Produkt ist eine Mischung von D-Mannitol und D-Sorbitol (25:75), da nur die Hälfte der D-Fructose zu D-Mannitol umgewandelt wird. Zur Trennung der beiden Zuckeralkohole sind aufwendige und teure Verfahren notwendig (Bhatt et al., 2013).

Biokatalytische Verfahren zur Herstellung von D-Mannitol sind ebenfalls beschrieben. So katalysieren Mannitol-Dehydrogenasen die Reduktion von D-Fructose zu D-Mannitol (US 7867740 B2; Kavanagh et al., 2002; Krahulec et al., 2011), aber auch fermentative Verfahren sind bekannt.

US 7358072 B2 beschreibt eine Fermentation im kontinuierlichen Fed-Batch-Verfahren mittels *Lactobacillus intermedius* NRRL B-3693, mit der aus D-Fructose (insgesamt 295 g) und D-Glucose (insgesamt 140 g, als sekundäre Kohlenstoffquelle) in 22 h 166 g/l D-Mannitol (was bei einem berechneten Volumen von 1,4 Liter 232 g D-Mannitol ergibt) produziert werden konnte. Insgesamt wurden nur ca. 53% der zugeführten Zucker (D-Fructose/D-Glucose) zu D-Mannitol umgesetzt.

Zucca et al. (2009) beschrieben zur Umsetzung von Cinnamaldehyd (Zimtaldehyd) zu Cinnamylalkohol (Zimtalkohol) in einem Substrat-gekoppelten Prozess mit einer Alkoholdehydrogenase aus *Saccharomyces cerevisiae,* wobei Ethanol oder 2-Propanol als oxidierbares Kosubstrat eingesetzt wurde. An die Reaktionsmischung wurde ein Luftstrom angelegt.

US 10113192 B2 beschreibt ein Verfahren zur Herstellung von D-Fructose aus D-Glucose. Im ersten Schritt wurde 2,5% (w/v) D-Glucose mittels einer Pyranose-2-Oxidase zu 2-Keto-D-glucose (D-Glucoson) oxidiert, wobei das entstandene Wasserstoffperoxid mit einer Katalase zu Wasser und Sauerstoff abgebaut wurde. Nach 24 h wurde die Reaktionsmischung aufgeheizt, um die Enzyme des ersten Schritts zu deaktivieren. Im Anschluss wurde der Reduktionsschritt zu D-Fructose mit einer Xylose-Reduktase aus *Candida tropicalis* durchgeführt - der benötigte Kofaktor NADPH wurde mit einer Alkoholdehydrogenase aus *Lactobacillus kefir* und 2-Propanol als Kosubstrat regeneriert. Die Reaktion wurde bei 30 °C unter kontinuierlichem Schütteln (850 rpm) ausgeführt. Es wird ferner ein offenes System verwendet, um die Verdampfung von Aceton zu ermöglichen und die Reaktion Richtung D-Fructose zu verschieben. Nach 6, 18 und 24 h wurde zusätzliches 2-Propanol nachdosiert. Auf diese Weise konnten 91% der D-Glucose zu D-Fructose umgesetzt werden, allerdings werden dazu 72 h (Schritt 1: 24 h, Schritt 2: 48 h) benötigt.

Hier setzt nun die vorliegende Erfindung an und möchte ein Verfahren zur Verfügung zu stellen, mit dem enzymatische Reduktionsreaktionen (Umwandlung einer organischen Keto-Verbindung wie D-Fructose in eine organische Hydroxy-Verbindung wie D-Mannitol) mit hohem Umsatz und in viel kürzerer Reaktionszeit bewerkstelligt werden können.

### Detaillierte Beschreibung der Erfindung

Diese Aufgabe wird bei einem Verfahren zur enzymatischen Reduktion einer organischen Keto-Verbindung zu einer organischen Hydroxy-Verbindung gelöst, indem NAD(P)H als Kofaktor der enzymatischen Reduktion eingesetzt wird und der durch die enzymatische Reduktion entstehende oxidierte Kofaktor NAD(P)⁺ enzymatisch mit 2-Propanol als Kosubstrat unter Bildung von Aceton zu NAD(P)H reduziert und Aceton durch Verdampfung abgetrennt wird und ist dadurch gekennzeichnet, dass die Verdampfung von Aceton in einer Dünnschichtbehandlungsvorrichtung vorgenommen wird.

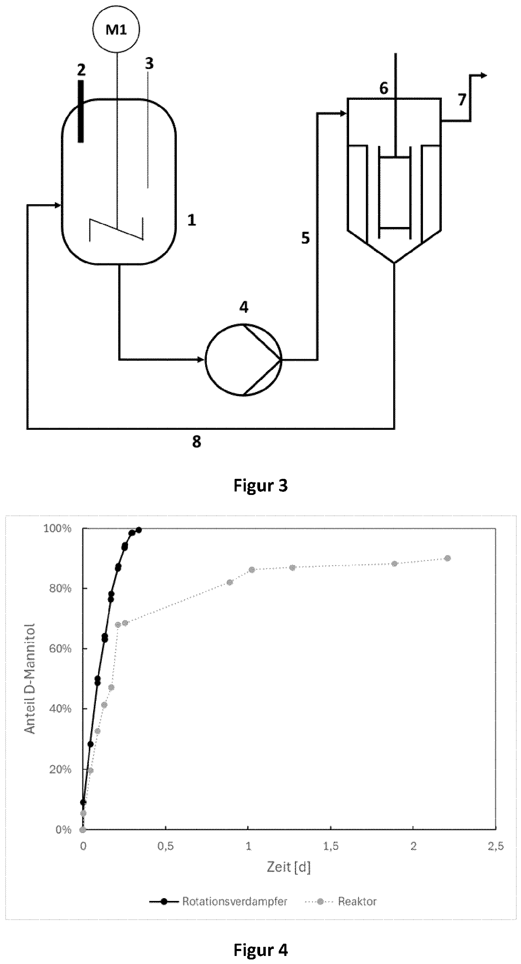

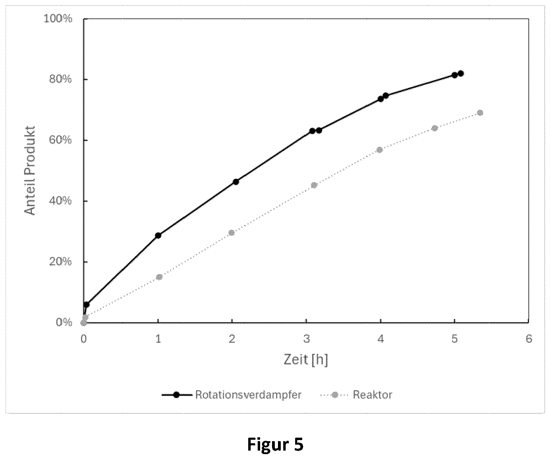

Es hat sich überraschenderweise gezeigt, dass im erfindungsgemäßen Verfahren nicht nur das Gleichgewicht der Regenerationsreaktion und damit der Hauptreaktion (Reduktion der organischen Keto-Verbindung) weiter in Richtung Produkt (organische Hydroxyverbindung) verschoben werden kann, sondern auch, dass die Reaktion in viel kürzerer Zeit abläuft (siehe Beispiele und Figuren 4 und 5).

Im Sinne dieser Anmeldung ist unter einer "organischen Keto-Verbindung" ein organisches Molekül zu verstehen, welches zumindest eine Keto-Gruppe (Keton oder Aldehyd) trägt. Unter einer "organischen Hydroxy-Verbindung" ist ein Molekül zu verstehen, welches zumindest eine Hydroxy- (Alkohol-)Gruppe aufweist.

Bevorzugte organische Keto-Verbindungen sind D-Fructose (Reduktionsprodukte D-Mannitol und D-Sorbitol), D-Glucose (Reduktionsprodukt D-Sorbitol, siehe US 9644227 B2 oder US 10253340 B2), 2- Keto-D-glucose/D-Glucoson (Reduktionsprodukte D-Fructose (siehe US 10113192 B2) und D-Mannose), D-Xylose (Reduktionsprodukt Xylitol, siehe US 9970038 B2), L-Arabinose (Reduktionsprodukt L-Arabitol), D-Galactose (Reduktionsprodukt D-Galactitol), D-Mannose (Reduktionsprodukt D-Mannitol), D-Psicose (Reduktionsprodukte D-Talitol und Allitol), Glycolaldehyd (Reduktionsprodukt Ethylenglycol), Glyceraldehyd (Reduktionsprodukt Glycerin, siehe WO 2024/100202 A1), Pyruvat (Reduktionsprodukt Lactat, siehe WO 2024/100202 A1), (3α,5β)-3-Hydroxy-7-oxocholan-24-säure/7-Ketolithocholsäure (Reduktionsprodukte (3α,5β,7α)-3,7-Dihydroxycholan-24-säure/Chenodesoxycholsäure und (3α,5β,7β)-3,7-Dihydroxycholan-24-säure/Ursodesoxycholsäure, siehe US 9644227 B2), (3α,5β,7α)-3,7-Dihydroxy-12-oxocholan-24-säure/12-Ketochenodesoxycholsäure (Reduktionsprodukte (3α,5β,7α,12α)-3,7,12-Trihydroxycholan-24-säure/Cholsäure und (3α,5β,7α,12β)-3,7,12-Trihydroxycholan-24-säure, siehe US 9644227 B2), (3α,5β,7β)-3,7-Dihydroxy-12-oxocholan-24-säure/12-Ketoursodesoxycholsäure (Reduktionsprodukte (3α,5β,7β,12α)-3,7,12-Trihydroxycholan-24-säure/Ursocholsäure und (3α,5β,7β,12β)-3,7,12-Trihydroxycholan-24-säure), (3α,5β,12α)-3,12-Dihydroxy-7-oxocholan-24-säure/7-Ketodesoxycholsäure (Reduktionsprodukte (3α,5β,7α,12α)-3,7,12-Trihydroxycholan-24-säure/Cholsäure und (3α,5β,7β,12α)-3,7,12-Trihydroxycholan-24-säure/Ursocholsäure), (3α,5β,12β)-3,12-Dihydroxy-7-oxocholan-24-säure (Reduktionsprodukte (3α,5β,7α,12β)-3,7,12-Trihydroxycholan-24-säure und (3α,5β,7β,12β)-3,7,12-Trihydroxycholan-24-säure), (3α,5β)-3-Hydroxy-7,12-dioxocholan-24-säure (Reduktionsprodukte (3α,5β,12α)-3,12-Dihydroxy-7-oxocholan-24-säure/7-Ketodesoxycholsäure, (3α,5β,12β)-3,12-Dihydroxy-7-oxocholan-24-säure, (3α,5β,7α)-3,7-Dihydroxy-12-oxocholan-24-säure/12-Ketochenodesoxycholsäure und (3α,5β,7β)-3,7-Dihydroxy-12-oxocholan-24-säure/12-Ketoursodesoxycholsäure, siehe US 9644227 B2), wobei D-Fructose und (3α,5β)-3-Hydroxy-7,12-dioxocholan-24-säure besonders bevorzugt sind.

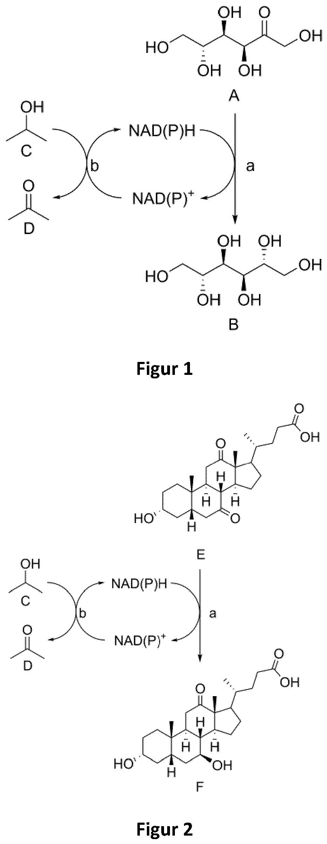

In den beiliegenden Figuren 1 und 2 sind die Reaktionsschemata von bevorzugten Ausführungsformen der erfindungsgemäßen Verfahren wiedergegeben. Die Bezeichnung A steht für D-Fructose, B für D-Mannitol, C für 2-Propanol, D für Aceton, E für (3α,5β)-3-Hydroxy-7,12-dioxocholan-24-säure und F für (3α,5β,7β)-3,7-Dihydroxy-12-oxocholan-24-säure/12-Ketoursodesoxycholsäure, a für Reduktion und b für Kofaktor-Regeneration.

Unter einer Dünnschichtbehandlungsvorrichtung ist im Sinne der vorliegenden Anmeldung eine Vorrichtung zu verstehen, die es ermöglicht, Aceton aus einer dünnen Flüssigkeitsschicht oder einem Flüssigkeitsfilm an einer beheizten Oberfläche (optional unter Anlegen von Unterdruck) abzudampfen.

Dabei kann es sich beispielsweise um einen Dünnschichtverdampfer, einen Fallfilmverdampfer oder einen Rotationsverdampfer, der im Labormaßstab besonders geeignet ist, handeln.

Dünnschicht- oder Fallfilmverdampfer haben sich im Bereich des Downstream Processing von biotechnologischen Verfahren (z.B. Fermentationen) bei der Aufkonzentrierung von verdünnten Produktlösungen bereits bewährt, wobei hohe Temperaturen hierbei oftmals vermieden werden müssen, um die Fermentationsprodukte nicht zu beeinträchtigen.

Beim Dünnschichtverdampfer wird die zu verdampfende Flüssigkeit mittels eines Rotors ausgerüstet mit Wischblättern als dünner Film an der beheizten Oberfläche des Gefäßes verteilt, wo die Verdampfung stattfindet. Beim Fallfilmverdampfer hingegen wird die zu verdampfende Flüssigkeit durch ein Bündel an beheizten Rohren geleitet, in denen die Verdampfung in einer dünnen Flüssigkeitsschicht stattfindet. In beiden Fällen kann je nach Anwendung zusätzlich ein Unterdruck angelegt werden (Dechow, 1989).

Die Erfindung betrifft auch eine Anlage zur Durchführung des Verfahrens, welche einen Reaktor und eine über eine Leitung mit dem Reaktor verbundene Dünnschichtbehandlungsvorrichtung umfasst, die über eine weitere Leitung mit dem Reaktor verbunden ist.

Eine bevorzugte Variante der Anlage zur Durchführung des Verfahrens ist in der beiliegenden Figur 3 schematisch dargestellt. Der Reaktor 1 ist mit einem Rührwerk M1, einem Aceton-Sensor 2 und weiterer Sensoren 3 wie pH-Elektrode, pO₂-Sonde, Manometer etc. ausgestattet. Zu Beginn der Reaktion beinhaltet der Reaktor 1 eine wässrige Reaktionslösung, welche ein Substrat, Zusätze wie Puffer, die zur Umsetzung des Substrats und etwaiger Zwischenstufen notwendigen Enzyme sowie eine Oxidoreduktase und 2-Propanol enthält. In der in Figur 1 dargestellten Reaktion wird D-Fructose mit NAD(P)H zu D-Mannitol reduziert und das dabei entstehende NAD(P)⁺ mittels 2-Propanol unter Bildung von Aceton regeneriert. Im Laufe der Reaktion wird somit durch Kofaktor-Regeneration mittels Oxidoreduktase Aceton aus 2-Propanol gebildet. Ein Anstieg des Aceton-Gehalts in der Reaktionslösung wird mittels Aceton-Sensor in Gasphase detektiert. Sobald ein je nach eingesetzten Enzymen festgelegter Schwellenwert (z.B. 2 v% in Lösung) festgestellt wird, wird die Reaktionslösung über Leitung 5 mittels einer Pumpe 4 in den Dünnschichtverdampfer 6 geführt, wo unter Vakuum das Aceton (und ein Teil des 2-Propanols und Wassers) abgedampft wird. Mit der Bezugsziffer 7 ist eine Leitung zur Entnahme des dampfförmigen Gemischs (Aceton mit kleinen Anteilen von 2-Propanol und Wasser) angedeutet, welches kondensiert und destillativ aufgearbeitet werden kann. Die abgereicherte Reaktionslösung wird über Leitung 8 zurück in den Reaktor 1 geführt.

Ein weiterer Vorteil der Entfernung von Aceton besteht darin, dass die Enzymaktivität nicht beeinträchtigt wird. So ist beispielsweise eine Alkoholdehydrogenase (Adh^{s}) der Fruchtfliege *Drosophila melanogaster* anfällig für nicht-kompetitive Produkt-Inhibierung durch Aceton (Winberg und McKinley-McKee, 1994). Durch die erfindungsmäßige Entfernung von Aceton aus der Reaktionsmischung kann eine Reduzierung oder Inhibierung der Enzymaktivität verhindert werden und die Reaktionsgeschwindigkeit gesteigert werden.

Festes D-Mannitol kann beispielsweise durch Kristallisation oder mittels Sprühtrocknung aus der wässerigen Lösung gewonnen werden. Eine chromatographische Abtrennung von Nebenprodukten oder des Substrats ist nicht notwendig.

Bei der Oxidoreduktase zur enzymatischen Reduktion von D-Fructose zu D-Mannitol handelt es sich bevorzugt um eine Mannitol-Dehydrogenase (NADH-abhängig: EC 1.1.1.67; NADPH-abhängig: EC 1.1.1.138) oder eine Sorbose-Reduktase (EC 1.1.1.289).

Die Oxidoreduktase zur Reduktion von D-Fructose zu D-Mannitol umfasst vorzugsweise eine Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls bzw. an ein Nukleinsäuremolekül mit der Nukleinsäuresequenz SEQ ID Nr. 1 bindet, wobei stringente Bedingungen vorzugsweise einen oder mehrere Waschschritte bei 65 °C und einer Salzkonzentration von 0,1x bis 2x SSC umfassen.

SEQ ID Nr. 1: 
ATGTCACACCCAACATCAGTTATTAACGAGCAGGTAGGCCCATTACCAACTAAGGCCCCACAACTTTCTA
AGAATGTGAATGATTTGTTCTCATTAAAGGGTAAGGTAGCTTCTGTCACCGGTTCCTCAGGAGGGATTG
GTTGGGCTGTAGCTGAAGCTTATGCCCAAGCAGGAGCAGACGTTGCTGTTTGGTACAATTCTAAAAAT
GCAGATGCTAAGGCTGAATACTTAACTAAGACTTACGGTGTGAAGTCGAAGGCATACAAGTGTAACAT
TTCTGATCCAGAAGACGTCGAAAAGGTAATTGGACAAATTGAAAAGGATTTCGGTACCATTGACGTTTT
CGTTGCCAATGCCGGTGTTCCATGGACAGAAGGTAGAAGTATTGAAGTTGAAGGATATGATTCGTGGA
AGAAGGTTATAGATTTGGACTTGAGTGGTGTCTACTATTGCGCTAAGGCTGTCGGAAAGATTTTCAAGA
AGAATGGTAAGGGTTCGCTTGTGTTCACAGCATCAATGTCTGGCCACATTGTGAATGTTCCACAATTGC
AAGCTCCATACAACGCCGCAAAGGCCGGTGTTTTGCACTTGAGTAAGTCATTAGCTGTCGAATGGGCCC
CATTCGCTAGAGTTAACACCATTTCACCTGGTTATATTGCCACAGAAATTTCCGACTTCGTTCCGGACGA
 
TGTCAAGGCTAAGTGGTGGCAATTAATTCCATTAGGAAGAGAAGCTCTTCCACAAGAATTAGTTGGTGC
TTACTTATATTTTGCATCCGATGCGTCTACTTACACTACAGGATCGGACTTATTAGTCGATGGTGGTTAC
TCTGCTCCATAA
SEQ ID Nr. 2:

MSHPTSVINEQVGPLPTKAPQLSKNVNDLFSLKGKVASVTGSSGGIGWAVAEAYAQAGADVAVWYNSKN
ADAKAEYLTKTYGVKSKAYKCNISDPEDVEKVIGQIEKDFGTIDVFVANAGVPWTEGRSIEVEGYDSWKKVID
LDLSGVYYCAKAVGKIFKKNGKGSLVFTASMSGHIVNVPQLQAPYNAAKAGVLHLSKSLAVEWAPFARVNTI
SPGYIATEISDFVPDDVKAKWWQLIPLGREALPQELVGAYLYFASDASTYTTGSDLLVDGGYSAP

Die Oxidoreduktase zur Reduktion von D-Fructose zu D-Mannitol umfasst oder besteht vorzugsweise aus einer Aminosäuresequenz, die eine Identität zu SEQ ID Nr. 2 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Reduktion von D-Fructose zu D-Mannitol die Aminosäuresequenz SEQ ID Nr. 2 oder besteht aus dieser.

Alternativ umfasst oder besteht die Oxidoreduktase zur Reduktion von D-Fructose zu D-Mannitol aus D-Fructose vorzugsweise aus einer Aminosäuresequenz, die durch eine Nukleinsäure kodiert wird, die eine Identität zu SEQ ID Nr. 1 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Reduktion von D-Fructose zu D-Mannitol kodiert, die Nukleinsäuresequenz SEQ ID Nr. 1 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990), verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 28, eine Erwartung (E) von 0,05, M = 1, N = -2 und einen Vergleich beider Stränge. Für Aminosäuresequenzen verwendet das blastp-Programm als Vorgaben eine Wortlänge von 3 und eine Erwartung (E) von 0,05 und die BLOSUM62-Scoring-Matrix (Henikoff & Henikoff, 1989), Alignments (B) von 50, Erwartung (E) von 0,05, M = 1, N = - 2.

Alternativ umfasst die Oxidoreduktase zur Reduktion von D-Fructose zu D-Mannitol vorzugsweise eine Aminosäuresequenz oder besteht aus dieser, die durch eine Nukleinsäure kodiert wird, die unter stringenten Bedingungen an einen komplementären Strang eines Nukleinsäuremoleküls bzw. an ein Nukleinsäuremolekül mit der Nukleinsäuresequenz SEQ ID Nr. 1 bindet. Wie hierin verwendet, beziehen sich die stringenten Bedingungen auf Bedingungen, unter denen sogenannte spezifische Hybride, jedoch keine unspezifischen Hybride gebildet werden.

Beispielsweise kann die Hybridisierung durch herkömmlich bekannte Verfahren durchgeführt werden, wie die von J. Sambrook et al. in Molecular Cloning, A Laboratory Manual, 2nd Ed., Cold Spring Harbor Laboratory (1989) beschriebenen. Unter stringenter Bedingung ist eine Bedingung gemeint, bei der das Waschen bei 65 °C und einer Salzkonzentration von 0,1x bis 2x SSC durchgeführt wird (wobei unter 1xSSC eine Mischung aus 0,15 M Natriumchlorid/0,015 M Natriumcitrat zu verstehen ist).

### Materialien

D-Fructose, NAD⁺, NADH-Dinatriumsalz, NADP⁺-Dinatriumsalz, NADPH-Tetranatriumsalz, Aceton, 2-Propanol und Acetonitril wurden von PanReac AppliChem (ITW Reagents), D-Glucose, D-Mannitol, IPTG (Isopropyl-β-D-thiogalactopyranosid) und Phosphorsäure wurden von Sigma-Aldrich, Kaliumdihydrogenphosphat, di-Kaliumhydrogenphosphat sowie Natriumdodecylsulfat (SDS) wurden von Carl Roth und Triethanolamin (TEA) wurde von Chem-Lab NV bezogen. (3α,5β)-3-Hydroxy-7,12-dioxocholan-24-säure wurde nach der Vorschrift von US 9644227 B2 hergestellt.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E*. *coli*-Zellen Top10F' transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optischen Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA)-HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymklassen und Spenderorganismen für die in den Beispielen verwendeten Enzyme.**

| **Enzymtyp (EC-Klasse)** | **katalysierte Reaktion(en)** | **Spenderorganismus** | **Literatur/Quelle** |
|---|---|---|---|
| Pyranose-2-Oxidase (P2O) | D-Glucose → D-Glucoson | *Coriolus sp.* | *(Sigma-Aldrich: P4234)* |
| Katalase | H₂O₂ → O₂ | *Aspergillus niger* | *(Sigma-Aldrich: C3515)* |
| Xylose-Reduktase (XR) | D-Glucoson → D-Fructose | *Candida tropicalis* | (US 10113192 B2) |
| Mannitol-Dehydrogenase (MDH) | D-Fructose → D-Mannitol | *Debaryomyces fabryi* | (NCBI Protein Database: XP_015465740.1)* |
| Alkoholdehydrogenase (ADH) | 2-Propanol → Aceton | *Lactobacillus kefir* | (US 10113192 B2) |
| 7β-Hydroxysteroid-Dehydrogenase | 7-Keto-Steroid → 7β-Hydroxysteroid | *Ruminococcus torques* | (US 9644227 B2) |

| | | | |
|---|---|---|---|
| * Anmerkung: Im NCBI-Datenbankeintrag ist die Mannitol-Dehydrogenase als Sorbose-Reduktase klassifiziert, allerdings wird die Region 27 bis 276 im Eintrag auch als "mannitol dehydrogenase (MDH)-like, classical (c) SDRs; cd05352" beschrieben. Die MDH-Aktivität von Sorbose-Reduktasen ist auch aus der Literatur bekannt (z.B. Sugisawa et al., 1991). | | | |

### Analytische Methoden

### High Performance Liquid Chromatography (HPLC)

Zur Quantifizierung von D-Glucose, D-Fructose, D-Glucoson und D-Mannitol mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Ca2+ (8%) Säule mit entsprechender Vorsäule verwendet und mit 3,5 v% 2-Propanol isokratisch eluiert (Flussrate 0,5 ml/min).

Zur Quantifizierung von (3α,5β)-3-Hydroxy-7,12-dioxocholan-24-säure und (3α,5β,7β)-3,7-Dihydroxy-12-oxocholan-24-säure wurde ebenfalls HPLC verwendet. Die Detektion erfolgte mittels eines UV-Detektors bei 200 nm. Zur Messung wurde eine Merck Purosphere^{®} STAR RP-18 endcapped (5µm) Säule verwendet, wobei die Elution mit einer Gradientenmethode aus Wasser (pH 2,6 mit Phosphorsäure) und Acetonitril erfolgte.

### Head Space Gas Chromatography (HS-GC)

Zur Quantifizierung von 2-Propanol und Aceton wurde ein Trace 1300 Gas-Chromatograph (Thermo Scientific) mit einem TriPlus 500 Headspace Autosampler und einer Supelco WATERCOL 1910 Säule (Länge 30 m, Durchmesser 0,25 mm, Filmdicke 0,2 µm) verwendet.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bioone Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67.10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Reduktion von D-Fructose zu D-Mannitol im Rotationsverdampfer

Die Reaktion wurde in einem Reaktor durchgeführt, wobei das Verdampfen von Aceton auf herkömmliche Art (Anlegen eines Luftstroms) sowie erfindungsgemäß in einem Dünnschichtverdampfer (im vorliegenden Beispiel ein Rotationsverdampfer) vorgenommen wurde.

Der Reaktoransatz wurde in einem Labfors 5 Tisch-Bioreaktor (Infors AG) durchgeführt. Dazu wurde ein Glasreaktor (Volumen 3,4 I) mit aufgesetztem Rührwerk und pH-Elektrode verwendet. Die pH-Kontrolle erfolgte durch die Zudosierung von 1M NaOH oder 1M H₂SO₄.

Zu Beginn wurden 207,4 ml einer D-Fructose-Lösung (482 g/l), 219,2 ml deionisiertes Wasser und 45,5 ml eines 200 mM TEA-HCl-Puffers (pH 8) im Reaktor vorgelegt und unter Rühren auf 30 °C gebracht.

Zum Start der Reaktion wurden 14,5 kU Mannitol-Dehydrogenase-Lysat, 12 kU Alkoholdehydrogenase-Lysat, 5 ml einer 5 mM NADP⁺-Lösung sowie 55 ml 2-Propanol eingebracht. Die Konzentration der D-Fructose zu Beginn der Reaktion betrug 200 g/l.

Beim Reaktor wurde ein Luftstrom von 0,15 l/min angelegt, um bei der Kofaktor-Regeneration gebildetes Aceton zu entfernen. Nach 12 h wurden 20 ml 2-Propanol nachdosiert.

Für den Versuch im Rotationsverdampfer wurde ein 1 I Rundkolben mit 207,4 ml einer D-Fructose-Lösung (482 g/l), 219,2 ml deionisiertes Wasser, 45,5 ml eines 200 mM TEA-HCl-Puffers (pH 8), 14,5 kU Mannitol-Dehydrogenase-Lysat, 12 kU Alkoholdehydrogenase-Lysat, 5 ml einer 5 mM NADP⁺-Lösung sowie 55 ml 2-Propanol befüllt. Die Konzentration der D-Fructose zu Beginn der Reaktion betrug 200 g/l.

Der Rundkolben wurde im Wasserbad auf 30 °C bei einem Druck von 50 mbar temperiert und eine Umdrehung von 210 rpm wurde eingestellt. Der Aceton- und 2-Propanol-Gehalt des im Auffangkolben erhaltenen Kondensats wurde mittels HS-GC bestimmt und die abgedampfte 2-Propanol-Menge wurde laufend ersetzt (die abgedampfte Aceton- und Wasser-Menge wurde mit deionisiertem Wasser ersetzt). Es wurde darauf geachtet, im Rundkolben keine höhere 2-Propanol-Konzentration als im Reaktor zu erreichen.

Während des Betriebs wurden laufend Proben von den Reaktionsansätzen genommen, die folgendermaßen analysiert wurden: 50 µl eines Ansatzes wurden mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen. Für die Bestimmung des Aceton- und 2-Propanol-Gehalts wurden 250 µl des Ansatzes mit 4,75 ml K₂CO₃-Lösung (0,2 g/ml) in einem HS-GC-Vial verdünnt und mittels HS-GC vermessen.

Die Konzentrationsverläufe sind in Figur 4 dargestellt. Sie zeigt, dass die Umsetzung im Rotationsverdampfer (schwarze durchgängige Linie) nicht nur wesentlich schneller abläuft als im Reaktor (hellgraue gepunktete Linie), sondern auch, dass das erfindungsgemäße Verfahren in nur 8 h zu einem vollständigen Umsatz führt, während im Reaktor selbst nach 53 h nur 90% der D-Fructose umgesetzt werden konnten.

### Beispiel 2

### Umwandlung von Invertzucker zu D-Mannitol

Der Reaktoransatz wurde in einem Labfors 5 Tisch-Bioreaktoren (Infors AG) durchgeführt. Dazu wurde ein Glasreaktor (Volumen 3,4 I) mit aufgesetztem Rührwerk, pO₂-Sensor und pH-Elektrode verwendet. Die pH-Kontrolle erfolgte durch die Zudosierung von 1M NaOH oder 1M H₂SO₄.

Zu Beginn wurden 197,4 ml einer Invertzucker-Lösung (D-Glucose/D-Fructose 1/1; 507 g/l), 201,0 ml deionisiertes Wasser und 42,7 ml eines 500 mM TEA-HCl-Puffers (pH 8) im Reaktor vorgelegt und unter Rühren auf 30 °C gebracht.

Zum Start der Reaktion wurden 10 ml eines Lysats enthaltend Pyranose-2-Oxidase und Katalase, 6 kU Xylose-Reduktase-Lysat, 16,1 kU Mannitol-Dehydrogenase-Lysat, 34 kU Alkoholdehydrogenase-Lysat sowie 55 ml 2-Propanol eingebracht. Die Konzentration des Invertzuckers zu Beginn der Reaktion betrug 200 g/l.

Es wurde ein Luftstrom von 0,05 l/min und ein Überdruck von 320 mbar angelegt.

Während des Betriebs wurden laufend Proben von der Reaktorlösung genommen, die folgendermaßen analysiert wurden: 50 µl des Ansatzes wurden mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Nach 2 h Laufzeit der gesamte Reaktorinhalt in einen 1 Liter-Rundkolben überführt. Am Rotationsverdampfer wurden bei 30 °C und 50 mbar Druck sowie einer Umdrehung von 210 rpm Aceton (und 2-Propanol) 60 min lang abgedampft. Danach wurde der Verlust mit deionisiertem Wasser bis zu einem Volumen von 500 ml ausgeglichen und 30 ml 2-Propanol wurden zugesetzt. Dieses Prozedere wurde nach 5 h und 22 h wiederholt.

Nach 26 h konnten 93% des Invertzuckers zu D-Mannitol umgesetzt werden.

Das Beispiel zeigt, dass das erfindungsgemäße Verfahren auf diese Weise auch in Kombination mit einer O₂-abhängigen Oxidationsreaktion (D-Glucose → D-Glucoson mit P2O) durchgeführt werden kann.

### Beispiel 3

### Reduktion von (3α,5β)-3-Hydroxy-7,12-dioxocholan-24-säure zu (3α,5β,7β)-3,7-Dihydroxy-12-oxocholan-24-säure

Die Reaktion wurde in einem Reaktor durchgeführt, wobei das Verdampfen von Aceton auf herkömmliche Art (Anlegen eines Luftstroms) sowie erfindungsgemäß in einem Dünnschichtverdampfer (im vorliegenden Beispiel ein Rotationsverdampfer) vorgenommen wurde.

Der Reaktoransatz wurde in einem Labfors 5 Tisch-Bioreaktor (Infors AG) durchgeführt. Dazu wurde ein Glasreaktor (Volumen 3,4 I) mit aufgesetztem Rührwerk und pH-Elektrode verwendet. Die pH-Kontrolle erfolgte durch die Zudosierung von 1M NaOH oder 1M H₂SO₄.

Zu Beginn wurden 500 ml einer Lösung von (3α,5β)-3-Hydroxy-7,12-dioxocholan-24-säure (50 g/l) in 50 mM TEA-HCl-Puffer (pH 8,4) unter Rühren auf 25 °C gebracht.

Zum Start der Reaktion wurden 3,25 kU 7β-Hydroxysteroid-Dehydrogenase-Lysat, 2,5 kU Alkoholdehydrogenase-Lysat, 10 mg NADP⁺ sowie 40 ml 2-Propanol eingebracht.

Beim Reaktor wurde ein Luftstrom von 2 l/min angelegt, um bei der Kofaktor-Regeneration gebildetes Aceton zu entfernen.

Für den Versuch im Rotationsverdampfer wurde ein 1l Rundkolben mit 500 ml einer Lösung von (3α,5β)-3-Hydroxy-7,12-dioxocholan-24-säure (50 g/l), 3,25 kU 7β-Hydroxysteroid-Dehydrogenase-Lysat, 2,5 kU Alkoholdehydrogenase-Lysat, 10 mg NADP⁺ sowie 40 ml 2-Propanol befüllt.

Der Rundkolben wurde im Wasserbad auf 25 °C bei einem Druck von 50 mbar temperiert und eine Umdrehung von 210 rpm wurde eingestellt. Der Aceton- und 2-Propanol-Gehalt des im Auffangkolben erhaltenen Kondensats wurde mittels HS-GC bestimmt und die abgedampfte 2-Propanol-Menge wurde laufend ersetzt (die abgedampfte Aceton- und Wasser-Menge wurde mit deionisiertem Wasser ersetzt). Es wurde darauf geachtet, im Rundkolben keine höhere 2-Propanol-Konzentration als im Reaktor zu erreichen.

Während des Betriebs wurden laufend Proben von den Reaktionsansätzen genommen, die folgendermaßen analysiert wurden: 150 µl des Ansatzes wurden mit 750 µl Lösungsmittelmischung (Acetonitril/H₂O/50% H₃PO₄ (90 + 9 + 1)) versetzt und im Eppendorf Thermomixer bei 55 °C und 1200 rpm für 15 min inkubiert. Die Probe wurde für 5 min bei max. g zentrifugiert. 100 µl des Überstands wurden mit 900 µl Lösungsmittelmischung im HPLC-Vial verdünnt und mittels HPLC (UV-Detektion) vermessen. Für die Bestimmung des Aceton- und 2-Propanol-Gehalts wurden 250 µl des Ansatzes mit 4,75 ml K₂CO₃-Lösung (0,2 g/ml) in einem HS-GC-Vial verdünnt und mittels HS-GC vermessen.

Die Konzentrationsverläufe sind in Figur 5 dargestellt. Sie zeigt, dass die Umsetzung im Rotationsverdampfer (schwarze durchgängige Linie; 81% Umsatz in 5 h) wesentlich schneller abläuft als im Reaktor (hellgraue gepunktete Linie; 66% Umsatz in 5 h).

### Literatur

Sellés Vidal, L., Kelly, C. L., Mordaka, P. M., & Heap, J. T. (2018). Review of NAD(P)H-dependent oxidoreductases: Properties, engineering and application. BBA - Proteins and Proteomics, 1866(2), 327-347. https://doi.org/10.1016/j.bbapap.2017.11.005
Chenault, H. K., Simon, E. S., & Whitesides, G. M. (1988). Cofactor regeneration for enzyme-catalysed synthesis. Biotechnology & Genetic Engineering Reviews, 6(1), 221-270. https://doi.org/10.1080/02648725.1988.10647849
Wang, X., Saba, T., Yiu, H. H. P., Howe, R. F., Anderson, J. A., & Shi, J. (2017). Cofactor NAD(P)H Regeneration Inspired by Heterogeneous Pathways. Chem, 2(5), 621-654. https://doi.org/10.1016/j.chempr.2017.04.009
Saha, B. C., & Racine, F. M. (2011). Biotechnological production of mannitol and its applications. Applied Microbiology and Biotechnology, 89, 879-891. https://doi.org/10.1007/s00253-010-2979-3
Bhatt, S. M., Mohan, A., & Srivastava S. K. (2013). Challenges in enzymatic route of mannitol production. ISRN Biotechnology, 2013, 914187. https://onlinelibrary.wiley.com/doi/full/10.5402/2013/914187
Chen, M., Zhang, W., Wu, H., Guang, C., & Mu, W. (2020). Mannitol: physiological functionalities, determination methods, biotechnological production, and applications. Applied Microbiology and Biotechnology, 104, 6941-6951. https://doi.org/10.1007/s00253-020-10757-y
Kavanagh, K. L., Klimacek, M., Nidetzky, B., & Wilson, D. K. (2002). Crystal structure of Pseudomonas fluorescens mannitol 2-dehydrogenase binary and ternary complexes. Specificity and catalytic mechanism. Journal of Biological Chemistry, 277(45), 43433-43442. https://doi.org/10.1074/jbc.M206914200
Krahulec, S., Armao, G. C., Klimacek, M., & Nidetzky, B. (2011). Enzymes of mannitol metabolism in the human pathogenic fungus Aspergillus fumigatus - kinetic properties of mannitol-1-phosphate 5-dehydrogenase and mannitol 2-dehydrogenase, and their physiological implications. FEBS Journal, 278(8), 1264-1276. https://doi.org/10.1111/j.1742-4658.2011.08047.x
Zucca, P., Littarru, M., Rescigno, A., & Sanjust, E. (2009). Cofactor Recycling for Selective Enzymatic Biotransformation of Cinnamaldehyde to Cinnamyl Alcohol. Bioscience, Biotechnology and Biochemistry, 73(5), 1224-1226. https://doi.org/10.1271/bbb.90025
Dechow, F. J. (1989). Separation and Purification Techniques in Biotechnology. Park Ridge, NJ, USA: Noyes Publications.
Winberg, J.-O., & McKinley-McKee, J. S. (1994). Drosophila melanogaster alcohol dehydrogenase: product-inhibition studies. Biochemical Journal, 301(3), 901-909. https://doi.org/10.1042/bj3010901
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Protein [Internet]. Bethesda (MD): National Library of Medicine (US), National Center for Biotechnology Information. Accession No. XP_015465740.1, Sorbose reductase SOU1 [Debaryomyces fabryi]. Verfügbar unter: https://www.ncbi.nlm.nih.gov/protein/XP_015465740.1
Sugisawa, T., Hoshino, T., & Fujiwara, A. (1991). Purification and Properties of NADPH-Linked L-Sorbose Reductase from Gluconobacter melanogenus N44-1. Agricultural and Biological Chemistry, 55(8), 2043-2049. https://doi.org/10.1080/00021369.1991.10870899

## Patentansprüche

1. Verfahren zur enzymatischen Reduktion einer organischen Keto-Verbindung zu einer organischen Hydroxy-Verbindung, wobei NAD(P)H als Kofaktor der enzymatischen Reduktion eingesetzt wird und der bei der Reduktion entstehende oxidierte Kofaktor NAD(P)⁺ enzymatisch mit 2-Propanol als Kosubstrat unter Bildung von Aceton zu NAD(P)H reduziert und das Aceton durch Verdampfung abgetrennt wird,
**dadurch gekennzeichnet, dass**
die Verdampfung in einer Dünnschichtbehandlungsvorrichtung vorgenommen wird.

2. Verfahren nach Anspruch 1 **dadurch gekennzeichnet, dass** die organische Keto-Verbindung D-Fructose und die organische Hydroxy-Verbindung D-Mannitol ist.

3. Verfahren nach Anspruch 1 **dadurch gekennzeichnet, dass** die organische Keto-Verbindung (3α,5β)-3-Hydroxy-7,12-dioxocholan-24-säure und die organische Hydroxy-Verbindung (3α,5β,7β)-3,7-Dihydroxy-12-oxocholan-24-säure ist.

4. Anlage zur Durchführung des Verfahrens nach einem der Ansprüche 1 bis 3, umfassend einen Reaktor (1), eine über eine Leitung (5) mit dem Reaktor (1) verbundenen Dünnschichtbehandlungsvorrichtung (6), die über eine Leitung (8) mit dem Reaktor (1) verbunden ist.

5. Verfahren nach Anspruch 2, **dadurch gekennzeichnet, dass** die Reduktion von D-Fructose zu D-Mannitol mit einer Oxidoreduktase durchgeführt wird, die vorzugsweise eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 1 bindet.
